# EUROPÄISCHE PATENTSCHRIFT

(11) **EP 2 432 557 B1**
(45) Veröffentlichungstag und Bekanntmachung des Hinweises auf die Patenterteilung: **16.04.2014**
(21) Anmeldenummer: 09763849.8
(22) Anmeldetag: 20.11.2009
(51) Int. Cl.: A61Q 5/00, A61Q 5/02, A61Q 5/12, A61K 8/89, A61K 8/898

(54) **KOSMETISCHES HAARSHAMPOO MIT EINEM ORGANOPOLYSILOXANMODIFIZIERTEM SACCHARID UND EINEM AMINOSILIKON**
COSMETIC HAIR SHAMPOO COMPRISING AN ORGANOPOLYSILOXANE-MODIFIED SACCHARIDE AND AN AMINO SILICONE
SHAMPOOING COSMÉTIQUE CONTENANT UN SACCHARIDE À MODIFICATION ORGANOPOLYSILOXANE ET UNE SILICONE AMINÉE

(30) Priorität: 08.12.2008 DE 102008060658
(43) Veröffentlichungstag der Anmeldung: 28.03.2012
(73) Patentinhaber: Beiersdorf AG, 20245 Hamburg (DE)
(72) Erfinder: SALADIN, Sandra, 20144 Hamburg (DE); MAHADESHWAR, Anand, Ramchandra, 22529 Hamburg (DE); KOHUT, Michaela, 20255 Hamburg (DE)
(86) Internationale Anmeldenummer: PCT/EP2009/008283
(87) Internationale Veröffentlichungsnummer: WO 2010/075913

(56) Entgegenhaltungen:
- EP-A1- 1 550 688
- EP-B1- 1 446 440
- WO-A1-2008/046763
- WO-A1-2009/019896
- WO-A1-2009/052272
- WO-A1-2009/079610
- WO-A2-2006/127883
- JP-A- 7 041 417
- JP-A- 63 139 106

## Beschreibung

Haarspülungen werden zur Pflege nach der Reinigung der Haare angewendet. Sie sind normalerweise auf Basis von kationischen Tensiden wie beispielsweise Cetyl Trimethyl Ammonium Chlorid und Fettalkoholen wie Cetyl und/oder Stearylalkohol. Allein reicht diese Kombination jedoch nicht für eine geeignete konditionierende Pflege an Haaren wie unter anderem Geschmeidigkeit, Kämmbarkeit und Glanz aus. Aus diesem Grund werden häufiger Silikone wie beispielsweise langkettige Polydimethylsiloxane sowie auch je nach Schädigungszustand der Haare substantivere Silikone wie beispielsweise Aminosilikone eingesetzt. Es besteht weiterhin Bedarf nach Haarfaserbehandlungsmitteln, die eine avivierende Wirkung am Haar haben und auch eine gute biologische Abbaubarkeit aufweisen. Weiterhin sollen die in solchen Mitteln eingesetzten Substanzen in möglichst geringen Mengen eine ausreichende Wirksamkeit entfalten um die Umwelt zu schonen. Hierfür bieten zuckerbasierte Siloxane mehrere Vorteile an wie z. B. Wasserstoffbrückenbildungen durch die hohe Anzahl an Hydroxyl-Gruppen, Feuchtigkeitsspeichern und Geschmeidigkeit auf Oberflächen.

Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar. Ein Nachteil der zuckerbasierten Siloxane liegt in einer leicht klebrigen Sensorik auf der nassen Haarsträhne bei der Spülungsapplikation und beim Ausspülen. Silikone, die Aminogruppen enthalten, stellen eine substantive Klasse der Silikone dar. In einer Spülungsformulierung bei pH-Werten von 4-5 sind diese Gruppen protoniert, so dass die kationische Natur des Silikons zu einer erhöhten Konditionierleistung führt. Jedoch ist die Kernseite, die Eigenschaft zur Neigung zur statischen Aufladung der Haare wenn die Silikone alleine eingesetzt werden und somit zum "Fly-Away" Effekt beim Verbraucher.

Der Stand der Technik kennt nach EP-1446440B1 über Saccharidreste funktionalisierte Organopolysiloxane und nach EP-1885331A2 den Einsatz von Saccharid-Siloxancopolymere in Personal-Care-Anwendungen. EP-1550688A1 beschreibt die Herstellung von organopolysiloxanmodifizierten Polysacchariden. EP-1097703B1 offenbart die Verwendung von Zuckersuspensionen und EP-1213316B1 kennt die kosmetische Verwendung von mit mehrwertigem Alkohol modifizierten Siloxanen. US-6066727 beschreibt die Herstellung von funktionalisierten Polysacchariden. WO-2008046763 A beschreibt die Verwendung von Siloxan-Saccharid-Copolymeren in der Kosmetik.

Es hat sich gezeigt, dass ein kosmetisches Haarbehandlungsmittel mit einem organopolysiloxanmodifiziertem Saccharid (A) und einem Aminosilikon (B) wobei (A) eine Molmasse von 22000 bis 27000 g/mol hat und die Struktur Z-S-Z aufweist wobei Z ein Monosaccharid oder dessen Derivat mit 6 oder 7 Kohlenstoffatomen ist, S ein Oligodimethylsiloxan, das terminal mit den Saccharidresten über die Gruppierung (CH2)3NH(CH2)2NH über eine Amidbindung verknüpft ist, den Stand der Technik verbessert.

Die Kombination von einem zuckerbasierten Silikon und von einem Aminosilikon führt zu einer erhöhten Pflegeleistung und zur besseren Kontrolle und Frisierbarkeit ohne den Nachteil der klebrigen Sensorik von den zuckerbasierten Silikonen und ohne eine statische Aufladung der Haare. Ein zuckerbasiertes Silikon mit einer für diese Erfindung geeignete Stuktur ist in CTFA als Gluconamidoethylaminopropylsilicone mit dem von Dow Corning angebotenen Handelsnamen Dow Corning CE-8810 Sugar Silicone Emulsion verfügbar. Die Zubereitung enthält mindestens ein anionisches Tensid, also ein Shampoo sein. Es ist von Vorteil, wenn das Aminosilikon (B) gewählt wird unter kationischen Emulsionen (insbesondere Dow Corning 939, 949, 959, Momentive Silikone SM2125 und SM 2658), kationischen Makroemulsionen (insbesondere Wacker Belsil ADM 6057 E), nichtionischen Makroemulsionen (insbesondere Wacker Belsil ADM 6060), nichtionischen Microemulsionen (insbesondere Wacker Belsil ADM 8020VP). Das Aminosilikon kann als Emulsion oder auch als Fluid vorliegen. Es ist von Vorteil, wenn das organopolysiloxanmodifizierte Saccharid (A) in Gehalten von 0.10 bis 2.5 Gew.-%, bevorzugt 1.0 bis 2.0 Gew.-%, jeweils Aktivgehalt bezogen auf das Gesamtgewicht der Zubereitung, enthalten ist. Besonders geeignet sind Gehalte von 1,5 Gew.-% für (A) und (B). Bevorzugt ist es wenn der Gehalt an (B) wenigstens so groß ist wie der Gehalt an (A). Besonders bevorzugt ist es, wenn das Verhältnis der Gehalte an (B) zu (A) 1:1 bis 1:3 ist, bevorzugt 1:1 bis 1:2. Durch derart überschüssiges Aminosilikon wird die Klebrigkeit der Zubereitung weiter verringert.

Es ist von Vorteil, wenn der pH-Wert der Zubereitung zwischen 3,5 und 4,1 liegt. Höhere pH-Werte führen zu einer verringerten Pflegeleistung, höhere zu einer verringerten Wirksamkeit einer Konservierung.

Es ist von Vorteil, wenn Z eine Heptose oder an die Gruppierung (CH2)3NH(CH2)2NH amidisch gebunden Glucuronsäure ist. Bevorzugt ist es, wenn Z eine Heptose ist, bevorzugt Sedoheptulose, Manno-heptulose, Allo-heptulose, Talo-heptulose, Manno-heptose oder Glucoheptose. Besonders bevorzugt ist es, wenn Z eine Hexose ist, bevorzugt Glucose, Mannose, Galactose, Fructose oder Sorbose.

### Details & Ergebnisse¹:

Von einem Expertenteam (n= 3) wurden zunächst 3 haarkonditionierende Formeln sensorisch bewertet. Formeln 1 bis 3 unterscheiden sich im Silikontyp. Formel 1 enthält eine gängige Aminosilikonemulsion, Formel 2 ein zuckerbasiertes Siloxan und Formel 3 eine Mischung der Silikone. Es wurde gefunden, dass Formel 3 während des Auftragens deutlich gehaltvoller ist als Formel 1 und besser gleitend als Formel 2. Formel 3 zeigte auch die beste Nasskämmbarkeit der Reihe. Diese Ergebnisse zugunsten der Formel 3 konnten von dem Expertenteam auch im trockenen Haar bestätigt werden.

"Gehaltvoll" im Sinne dieser Schrift wird wie folgt festgelegt: Es wird gemessen, in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleich bleibendem Druck an der Haarsträhne von oben nach unten. Hierbei wird eine 10er Skala verwendet bei der 10 das höchstmögliche Gleitvermögen darstellt.

"Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

Shampooformulierungen im Sinne dieser Erfindung sind hiermit abgebildet:
¹ Alle im Text genannten Angaben sind Aktivgehalte.

### Shampoos

### 1) Conditioner-Shampoo

| | 1 | 2 | 3 | 4 | 5 | 6 | 7 |
|---|---|---|---|---|---|---|---|
| Natrium Laurylethersulfat | 7 | 7 | 8 | 9 | 9 | 10 | 12 |
| Cocamidopropyl Betain | - | 3 | 3.5 | 3.5 | 4 | 4 | 3 |
| Di-Natrium PEG-5 Laurylcitrat Sulfosuccinat | - | - | 2 | 3 | 3 | - | - |
| Decyl Glucoside | - | 2 | -3 | - | - | - | - |
| Cocamide DEA | - | - | 0.5 | 3 | - | - | 2.5 |
| Natrium Cocoamphoacetat | 3 | - | - | - | - | - | - |
| Guar Hydroxypropyltrimonium Chloride | - | 0.2 | 0.25 | 0.3 | - | 0.4 | - |
| Polyquaternium-10 | - | - | 0.2 | - | 0.1 | - | 0.3 |
| Glucoamidoethylaminopropylsilicone | 0,2 | 1,1 | 0,3 | 1,7 | 1,1 | 0,6 | 0,8 |
| Amodimethicone + Trideceth-12 + Cetrimoniumchloride (DC 939) | 0,9 | 1,6 | 0,5 | 2,5 | 2,5 | 1,3 | 1,9 |
| PEG-200 Hydrogenated Glyceryl Palmate | - | - | 0,2 | - | - | 1,0 | - |
| PEG-40 hydrogeniertes Rizinusöl | 0.2 | 0.3 | 0.4 | 0,5 | 0.6 | 0,5 | 0.5 |
| PEG-3 Distearat | 1.5 | - | 0.75 | 1.5 | 1.0 | - | 0.5 |
| Glycol Distearat | - | 4.0 | | -- | - | - | - |
| PEG-7 Glycerylcocoat | 0.5 | 1 | 2 | - | - | - | - |
| Natrium Salicylat | 0.2 | 0.3 | 0.4 | 0.4 | 0.4 | - | - |
| Natrium Benzoat | 0.5 | 0.4 | 0.3 | 0.2 | 0.4 | - | 0.5 |
| Parfüm | 0,3 | 0,5 | 0,3 | 0,3 | 0,4 | 0,7 | 0,3 |
| Benzophenon-4 | 0,2 | 0,25 | - | 0,3 | - | - | - |
| Oryzanol | - | - | - | 0,5 | - | - | 0,05 |
| Piroctone Olamine | - | - | - | - | - | 0.1 | 0.2 |
| Niacinamid | - | 0.01 | - | - | - | - | - |
| Panthenol | - | - | 0.01 | - | - | - | 0.01 |
| Jojobaöl | - | - | - | - | 0,1 | - | - |
| Parfüm | 0.5 | 0.8 | - | 0.7 | 0.5 | 0.6 | 0.9 |
| Natronlauge | q.s. | q.s. | q.s. | q.s. | q.s. | q.s. | - |
| Citronensäure | q.s. | q.s. | q.s. | q.s. | q.s. | q.s. | q.s. |
| Wasser | ad 100 | ad 100 | ad 100 | ad 100 | ad 100 | ad 100 | ad 100 |

### 2) Conditioner-Shampoo mit Perlglanz (und Trübungsmittel)

| | **1** | **2** | **3** | **4** | **5** | **6** |
|---|---|---|---|---|---|---|
| Natrium Laurylethersulfat | 9 | 9 | 9 | 9 | 9 | 9 |
| Cocamidopropyl Betain | 4 | 3 | 4 | 3 | 4 | 3 |
| Dinatrium PEG-5 Laurylcitrat Sulfosuccinat | 3 | 3 | 3 | 2 | 3 | 4 |
| PEG-200 Hydrogenated Glyceryl Palmate | - | - | 0.1 | - | 0,3 | 0,4 |
| Guar Hydroxypropyltrimonium Chloride | - | 0.2 | 0.25 | 0.3 | - | 0.4 |
| Polyquaternium-10 | - | - | 0.2 | - | 0.1 | - |
| Glucoamidoethylaminopropylsilicone | 1,1 | 1,1 | 0,8 | 1,1 | 0,6 | 0,6 |
| Amodimethicone + Trideceth-12 + Cetrimoniumchloride (DC 939) | 1, 6 | 3,1 | 2,5 | 1,9 | 2,2 | 1,4 |
| PEG-200 Hydrogenated Glyceryl Palmate | - | - | 0,2 | - | - | 1,0 |
| Glycol Distearat | - | - | - | - | 0.5 | 0,5 |
| PEG-40 hydriertes Rizinusöl | 0.2 | 0.3 | 0.4 | 0.5 | 0.2 | 0,3 |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| Zitronensäure | q.s. | q.s. | q.s. | q.s. | q.s. | q.s. |
| Parfüm | 0.5 | 0.8 | - | 0.7 | 0.5 | 0.6 |
| Wasser | ad 100 | ad 100 | ad 100 | ad 100 | ad 100 | ad 100 |

### 3) Mildes Baby Shampoo

| | **1** | **2** | **3** | **4** | **5** | **6** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyceryl Palmate | - | - | - | 0.2 | 0.3 | 0,4 |
| Guar Hydroxypropyltrimonium Chloride | - | 0.2 | 0.25 | 0.3 | - | 0.4 |
| Polyquaternium-10 | - | - | 0.2 | - | 0.1 | - |
| Glucoamidoethylaminopropylsilicone | 0,4 | 1,1 | 0,4 | 0,6 | 3,8 | 1,0 |
| Amodimethicone + Trideceth-12 + Cetrimoniumchloride (DC 939) | 0,6 | 1,6 | 2,5 | 2,2 | 0,9 | 3,1 |
| PEG-3 Distearat | - | - | 0,5 | 2 | - | - |
| Glycol Distearat | - | - | - | - | 0.5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | 0.5 | 0.8 | - | 0.7 | 0.5 | 0.6 |
| Wasser | ad 100 | ad 100 | ad 100 | ad 100 | ad 100 | ad 100 |

### 4) Antischuppen Shampoo/Mildes Kopfhaut Shampoo

| | **1** | **2** | **3** | **4** | **5** | **6** |
|---|---|---|---|---|---|---|
| Natrium Laurylethersulfat | 9 | 9 | 9 | 9 | 10 | - |
| Natrium Myristylethersulfat | - | - | - | - | - | 6 |
| Cocamidopropyl Betain | 4 | 4 | 3 | 3 | 4 | 5 |
| Dinatrium PEG-5 Laurylcitrat Sulfosuccinat | 3 | 3 | 3 | 3 | - | - |
| Natrium Cocoamphoacetat | - | - | - | - | - | 2.5 |
| Decylglucosid | - | - | - | - | - | 2.5 |
| PEG-200 Hydrogenated Glyceryl Palmate | - | - | 0.1 | - | 0.4 | 3 |
| Guar Hydroxypropyltrimonium Chloride | - | 0.2 | 0.25 | 0.3 | - | 0.4 |
| Polyquaternium-10 | - | - | 0.2 | - | 0.1 | - |
| Glucoamidoethylaminopropylsilicone | 0,4 | 1,1 | 0,4 | 0,6 | 3,8 | 1,0 |
| Amodimethicone + Trideceth-12 + Cetrimoniumchloride (DC 939) | 0,6 | 1,6 | 2,5 | 2,2 | 0,9 | 3,1 |
| Climbazol | 0.5 | 0.5 | - | 0.5 | 1.0 | - |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| PEG-3 Distearat | 1.5 | 3 | 0,75 | 0,5 | - | - |
| Glycol Distearat | - | - | - | - | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | 0.5 | 0.8 | - | 0.7 | 0.5 | 0.6 |
| Wasser | ad 100 | ad 100 | ad 100 | ad 100 | ad 100 | ad 100 |

## Patentansprüche

1. Kosmetisches Haarshampoo mit einem organopolysiloxanmodifiziertem Saccharid (A) und einem Aminositikon (B) wobei (A) eine Molmasse von 22000 bis 27000 g/mol hat und die Struktur Z-S-Z aufweist wobei
Z ein Monosaccharid mit 6 oder 7 Kohlenstoffatomen oder eine Glucuronsäure ist,
S ein Oligodimethylsiloxan, das terminal mit den Saccharidresten über die Gruppierung (CH₂)₃NH(CH₂)₂NH über eine Amidbindung verknüpft ist,
**dadurch gekennzeichnet, dass**
die Zubereitung mindestens ein anionisches Tensid enthält.

2. Zubereitung nach Anspruch 1 **dadurch gekennzeichnet, dass** das Aminosilikon (B) gewählt wird unter kationischen Emulsionen Amodimethicon/Trideceth-12/Cetrimoniumchlorid (Dow Corning 939).

3. Zubereitung nach einem der vorangehenden Patentansprüche **dadurch gekennzeichnet, dass** 0.10 bis 2.5 Gew.-%, jeweils Aktivgehalt bezogen auf das Gesamtgewicht der Zubereitung, an organopolysiloxanmodifiziertem Saccharid (A) enthalten sind.

4. Zubereitung nach Anspruch 3 **dadurch gekennzeichnet, dass** 1.0 bis 2.0 Gew.-%, jeweils Aktivgehalt bezogen auf das Gesamtgewicht der Zubereitung, an organopolysiloxanmodifiziertem Saccharid (A) enthalten sind.

5. Zubereitung nach einem der vorangehenden Patentansprüche **dadurch gekennzeichnet, dass** der Gehalt an (B) wenigstens so groß ist wie der Gehalt an (A).

6. Zubereitung nach einem der vorangehenden Patentansprüche 1 bis 4 **dadurch gekennzeichnet, dass** das Verhältnis der Gehalte an (B) zu (A) 1:1 bis 1:3 ist, bevorzugt 1:1 bis 1:2.

7. Zubereitung nach einem der vorangehenden Patentansprüche **dadurch gekennzeichnet, dass** der pH-Wert der Zubereitung zwischen 3,5 und 4,1 liegt.

8. Zubereitung nach einem der vorangehenden Patentansprüche **dadurch gekennzeichnet, dass** Z eine Heptose oder an die Gruppierung (CH₂)₃NH(CH₂)₂NH amidisch gebundene. Glucuronsäure ist.

9. Zubereitung nach einem der vorangehenden Patentansprüche **dadurch gekennzeichnet, dass** Z eine Heptose ist, bevorzugt Sedoheptulose, Manno-heptulose, Allo-heptulose, Talo-heptulose, Manno-heptose oder Glucoheptose.

10. Zubereitung nach einem der Patentansprüche 1 bis 7 **dadurch gekennzeichnet, dass** Z eine Hexose ist, bevorzugt Glucose, Mannose, Galactose, Fructose oder Sorbose.

## Claims

1. Cosmetic hair shampoo with an organopolysiloxanemodified saccharide (A) and an aminosilicone (B), where (A) has a molar mass of from 22 000 to 27 000 g/mol and has the structure Z-S-Z, where Z is a monosaccharide having 6 or 7 carbon atoms or a glucuronic acid,
S is an oligodimethylsiloxane which is terminally linked with the saccharide radicals via the group (CH₂)₃NH(CH₂)₂NH via an amide bond,
**characterized in that**
the preparation comprises at least one anionic surfactant.

2. Preparation according to Claim 1, **characterized in that** the aminosilicone (B) is selected among cationic emulsions amodimethicone/trideceth-12/ cetrimonium chloride (Dow Corning 939).

3. Preparation according to one of the preceding patent claims, **characterized in that** 0.10 to 2.5% by weight, in each case active content based on the total weight of the preparation, of organopolysiloxane-modified saccharide (A) are present.

4. Preparation according to Claim 3, **characterized in that** 1.0 to 2.0% by weight, in each case active content based on the total weight of the preparation, of organopolysiloxane-modified saccharide (A) are present.

5. Preparation according to one of the preceding patent claims, **characterized in that** the content of (B) is at least as large as the content of (A).

6. Preparation according to one of the preceding patent Claims 1 to 4, **characterized in that** the ratio of the contents of (B) to (A) is 1:1 to 1:3, preferably 1:1 to 1:2.

7. Preparation according to one of the preceding patent claims, **characterized in that** the pH of the preparation is between 3.5 and 4.1.

8. Preparation according to one of the preceding patent claims, **characterized in that** Z is a heptose or glucuronic acid amidically bonded to the group (CH₂)₃NH(CH₂)₂NH.

9. Preparation according to one of the preceding patent claims, **characterized in that** Z is a heptose, preferably sedoheptulose, mannoheptulose, alloheptulose, taloheptulose, mannoheptose or glucoheptose.

10. Preparation according to one of patent Claims 1 to 7, **characterized in that** Z is a hexose, preferably glucose, mannose, galactose, fructose or sorbose.

## Revendications

1. Shampooing capillaire cosmétique comportant un saccharide (A) modifié avec un organopolysiloxane, et un aminosilicone (B), (A) ayant une masse moléculaire de 22 000 à 27 000 g/mole et présentant la structure Z-S-Z, où
Z est un monosaccharide ayant 6 ou 7 atomes de carbone ou un acide glucuronique,
S est un oligodiméthylsiloxane qui est lié en bout de chaîne aux radicaux saccharide par une liaison amide par l'intermédiaire du groupement (CH₂)₃NH(CH₂)₂NH, **caractérisé en ce que**
la préparation contient au moins un tensioactif anionique.

2. Préparation selon la revendication 1, **caractérisée en ce que** l'aminosilicone (B) est choisi parmi des émulsions cationiques amodimethicone/trideceth-12/chlorure de cétrimonium (Dow Corning 939).

3. Préparation selon l'une quelconque des revendications précédentes, **caractérisée en ce que** 0,10 à 2,5 % en poids, chaque fois en teneur en actif, de saccharide (A) modifié avec un organopolysiloxane, sont contenus par rapport au poids total de la préparation.

4. Préparation selon la revendication 3, **caractérisée en ce que** 1,0 à 2,0 % en poids, chaque fois en teneur en actif, de saccharide (A) modifié avec un organopolysiloxane, sont contenus par rapport au poids total de la préparation.

5. Préparation selon l'une quelconque des revendications précédentes, **caractérisée en ce que** la teneur en (B) est au moins aussi élevée que la teneur en (A).

6. Préparation selon l'une quelconque des revendications 1 à 4 précédentes, **caractérisée en ce que** le rapport des teneurs en (B) à (A) vaut de 1:1 à 1:3, de préférence de 1:1 à 1:2.

7. Préparation selon l'une quelconque des revendications précédentes, **caractérisée en ce que** le pH de la préparation est compris entre 3,5 et 4,1.

8. Préparation selon l'une quelconque des revendications précédentes, **caractérisée en ce que** Z est un heptose ou l'acide glucuronique lié par une liaison amide au groupement (CH₂)₃NH(CH₂)₂NH.

9. Préparation selon l'une quelconque des revendications précédentes, **caractérisée en ce que** Z est un heptose, de préférence le sédoheptulose, le manno-heptulose, l'allo-heptulose, le talo-heptulose, le manno-heptose ou le glucoheptose.

10. Préparation selon l'une quelconque des revendications 1 à 7, **caractérisée en ce que** Z est un hexose, de préférence le glucose, le mannose, le galactose, le fructose ou le sorbose.
